(19) **Europäisches Patentamt / European Patent Office / Office européen des brevets**

(11) **EP 1 860 461 B1**

(12) **EUROPEAN PATENT SPECIFICATION**

(45) Date of publication and mention
of the grant of the patent:
**03.12.2008 Bulletin 2008/49**

(51) Int Cl.:
**G01S 15/89** (2006.01)   **A61B 8/00** (2006.01)
**G10K 11/34** (2006.01)   **G01S 7/52** (2006.01)

(21) Application number: **07010211.6**

(22) Date of filing: **23.05.2007**

(54) **Ultrasound diagnostic system and method for forming IQ data without quadrature demodulator**

Ultraschalldiagnosesystem und Verfahren zur Bildung von IQ-Daten ohne Quadratur-Demodulator

Système et procédé de diagnostic à ultrason pour former des données QI sans démodulateur en quadrature

(84) Designated Contracting States:
**DE FR IT**

(30) Priority: **23.05.2006 KR 20060046253**
**17.11.2006 KR 20060114067**
**22.05.2007 KR 20070049607**

(43) Date of publication of application:
**28.11.2007 Bulletin 2007/48**

(73) Proprietor: **MEDISON CO., LTD.**
**Kangwon-do 250-870 (KR)**

(72) Inventors:
• **Bae, Moo Ho**
**Seoul 138-240 (KR)**
• **Daigle, Ronald E.**
**Washington 98053 (US)**
• **Ahn, Chi Young,**
**Discusser & Medison Building**
**Seoul 135-280 (KR)**

• **Yoon, Ra Young,**
**Discusser & Medison Building**
**Seoul 135-280 (KR)**

(74) Representative: **Lorenz, Werner**
**Lorenz & Kollegen**
**Patent- und Rechtsanwaltskanzlei**
**Alte Ulmer Strasse 2-4**
**89522 Heidenheim (DE)**

(56) References cited:
**WO-A-20/04064619**

• **RANGANATHAN K ET AL: "A prototype low-cost handheld ultrasound imaging system" PROCEEDINGS OF THE SPIE - THE INTERNATIONAL SOCIETY FOR OPTICAL ENGINEERING SPIE-INT. SOC. OPT. ENG USA, vol. 5373, no. 1, 2004, pages 24-32, XP002447113 ISSN: 0277-786X**

**Description**

BACKGROUND

1. Field

The present invention generally relates to an ultrasound diagnostic system, and more particularly to an ultrasound diagnostic system and a method for forming IQ data without a quadrature demodulator.

2. Background

An ultrasound diagnostic system has become an important and popular diagnostic tool since it has a wide range of applications. Specifically, due to its non-invasive and non-destructive nature, the ultrasound diagnostic system has been extensively used in the medical profession. The ultrasound is transmitted to a target object through a probe equipped in the ultrasound diagnostic system. Ultrasound echoes from the target object reach the probe. The ultrasound echoes are then converted into electrical receiving signals in analog-form. Ultrasound images are formed based on the electric receiving signals obtained from the ultrasound echoes.

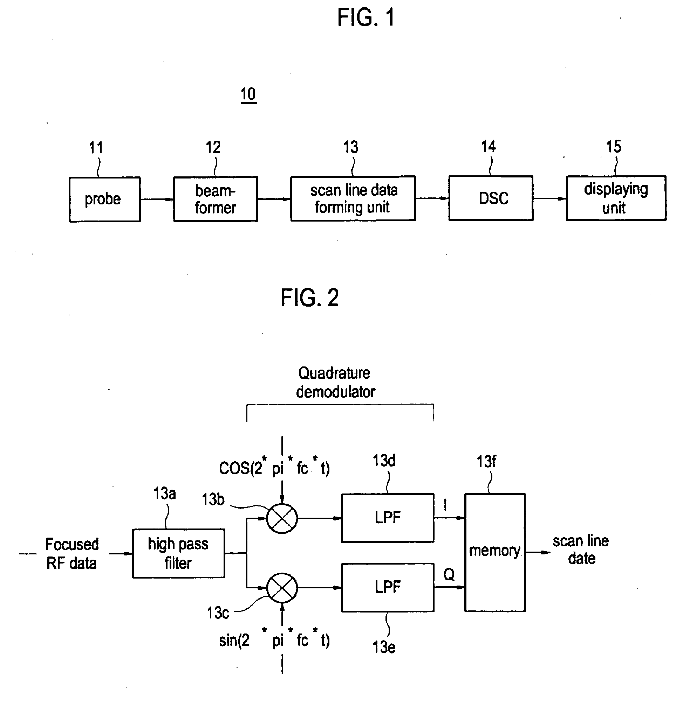

As shown in FIG. 1, a conventional ultrasound diagnostic system 10 includes a probe 11, a beam-former 12, a scan line data forming unit 13, a digital scan converter (DSC) 14 and a displaying unit 15. The probe 11 includes a plurality of probe elements, which convert electrical transmission signals into ultrasound transmission signals and transmit the ultrasound transmission signals to a target object. The probe elements also receive ultrasound echoes from the target object and convert the ultrasound echoes into electrical receiving signals in analog-form. The ultrasound echoes from the target object are inputted into the probe elements at different times due to the distance differences between the probe elements and target object. The beam-former 12 converts the analog receiving signals into digital signals, delays the digital signals in consideration of the arriving time of the ultrasound echoes to each probe element, and forms receiving focus signals (RF signals) by adding the delayed signals. The scan line data forming unit 13 converts the RF signals into base-band signals and forms scan line data. Referring to FIG. 2, the scan line data forming unit 13 includes a high pass filter 13a for removing a direct current (DC) component from the RF signals, a cosine-function multiplier 13b, a sine-function multiplier 13c, low pass filters (LPFs) 13d and 13e and a memory 13f. The cosine-function multiplier 13b, the sine-function multiplier 13c and the low pass filters 13d and 13e are provided with a quadrature demodulator.

The RF signals are inputted into the high pass filter 13a. Further, the cosine and sine functions are multiplied by the outputs of the high pass filter 13a in the cosine-function multiplier 13b and the sine-function multiplier 13c, respectively. The outputs from the multipliers 13b and 13c are inputted into the low pass filters 13d and 13e, respectively, demodulated base band signals, i.e., in-phase component data (I data) and quadrature-phase component data (Q data), can be obtained. The IQ data, which form the scan line data, are stored in the memory 36. The displaying unit 15 displays an ultrasound image with the scan line data, which have been scan-converted by the DSC. In FIG. 2, the "fc" denotes a center frequency.

In the conventional ultrasound diagnostic system, the quadrature demodulator must be equipped in order to form the IQ data. However, this causes certain limitations or restrictions in designing the system.

An ultrasound system for forming IQ data without a quadratur demodulator is disclosed in "A Prototype Low Cost Handheld Ultrasound Imaging System", Proc. of SPIE, vol. 5373, p. 24-32 (2004) by Ranganathan, K. et al. This system forms the closest prior art for the present invention. It includes a unit for direct sampled IQ beamforming. This unit comprises per ultrasound transducer element a stage with two sample and hold circuits, one each for the I and Q channels. The two channels are sampled apart at a quarter period of the center frequency of the received ultrasound signal ($1/4f_0$). The value of the sampling rate is not specified. The actual beamforming and focusing is performed after the forming of the I and Q signals.

BRIEF DESCRIPTION OF THE DRAWINGS

Arrangements and embodiments may be described in detail with reference to the following drawings in which like reference numerals refer to like elements and wherein:

FIG. 1 is a block diagram showing a conventional ultrasound diagnostic system;
FIG. 2 is a schematic diagram showing a conventional method of forming IQ data with a quadrature demodulator equipped in the conventional ultrasound diagnostic system;
FIG. 3 is a block diagram showing an ultrasound diagnostic system constructed in accordance with an embodiment of the present invention;
FIG. 4 is a block diagram showing a beam-former constructed in accordance with an embodiment of the present invention;

FIG. 5 is a schematic diagram showing a dual port RAM for delaying digital signals in accordance with the present invention;

FIG. 6 is a block diagram showing an extracting unit for controlling an amount of digital signals and an interpolating unit in accordance with the present invention;

FIG. 7 is an exemplary diagram showing extraction of high and low frequency signals in accordance with the present invention;

FIG. 8 is a graph showing a relationship between the amount of digital signals outputted from the ultrasound signals and a center frequency;

FIGS. 9 to 11 are schematic diagrams for illustrating a method of forming IQ data in accordance with the present invention; and

FIG. 12 is a block diagram showing an ultrasound diagnostic system constructed in accordance with another embodiment of the present invention.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

In the embodiments of the present invention, focused-receiving signals are formed at a rate of n-times of a center frequency of analog receiving signals, wherein "n" is a positive integer. Further, at least one pair of focused-receiving signals are selected to form IQ data without a quadrature demodulator. In each pair, the focused-receiving signals have a phase difference of $\lambda/4$, where the "$\lambda$" is a wavelength, which is defined with the center frequency of analog receiving signals.

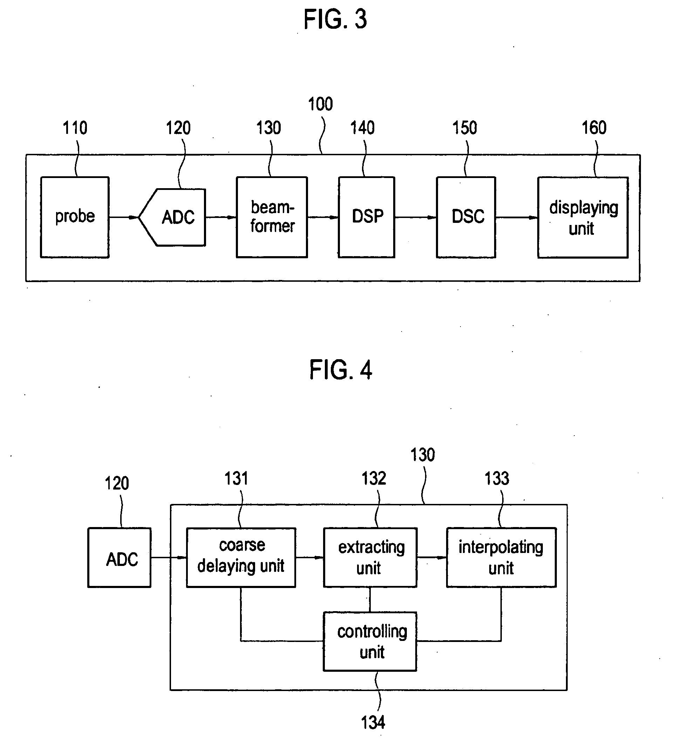

FIG. 3 is a block diagram showing an ultrasound diagnostic system constructed in accordance with an embodiment of the present invention. The ultrasound diagnostic system 100 includes a probe 110, an analog-digital converter (ADC) 120, a beam-former 130, a digital signal processor (DSP) 140, a digital scan converter (DSC) 150 and a displaying unit 160.

The probe 110 includes a plurality of probe elements. Each probe element converts electrical transmission signals into ultrasound transmission signals and transmits the ultrasound transmission signals to a target object. The probe element also receives ultrasound echoes from the target object and converts the ultrasound echoes into electrical receive signals of analog-form. The analog receiving signals outputted from the probe 110 have a center frequency, which reflects the characteristics of the probe 110 and internal tissues of the target object.

The ADC 120 forms digital signals from the analog receiving signals. The number of ADCs 120 is equal to that of probe elements. Further, the ADCs 120 correspond one-to-one with the probe elements. Each ADC samples the analog receiving signals, which are outputted from each probe element, at a predetermined sampling rate (e.g., 60 MHz), regardless of the center frequency of the analog signals, and converts the analog receiving signals into the digital signals. Therefore, the lower the center frequency of the analog signals is, the more the digital signals are obtained per one cycle, which is defined with the center frequency. Also, the higher the center frequency of the analog signals is, the less the digital signals are obtained per the cycle.

The beam-former 130 forms focused-receiving signals by extracting parts of the digital signals at a rate of n-times of the center frequency, wherein the "n" denotes a positive integer. Therefore, the beam-former 130 may provide a uniform amount of the focused-receiving signals. Specifically, the beam-former 130 delays the digital signals, which are obtained with the constant sample rate in the ADC 120, in consideration of the distances between the probe elements and the target object, and extracts parts of the delayed digital signals at the rate of n-times of the center frequency to control the amount of the delayed digital signals. It then forms the focused-receiving signals by interpolating the extracted digital signals. Information of the center frequency may be directly provided by a user such as a system designer or an operator. The ultrasound diagnostic system may further include a center frequency providing unit, which analyzes the analog receiving signals outputted from the probe elements and provides the information of the center frequency as a result of the analysis. Referring to FIG. 4, the beam-former 130 includes a coarse delaying unit 131, an extracting unit 132, an interpolating unit 133 and a controlling unit 134. Although a transmitting beam forming unit and the receiving beam forming unit are not shown in FIG. 4, it is natural that the beam-former 130 includes both units in order to perform its basic functions. Further, the beam-former may include a gain controlling unit for compensating attenuation of signals. The controlling unit 134 controls the coarse delaying unit 131, the extracting unit 132 and the interpolating unit 133.

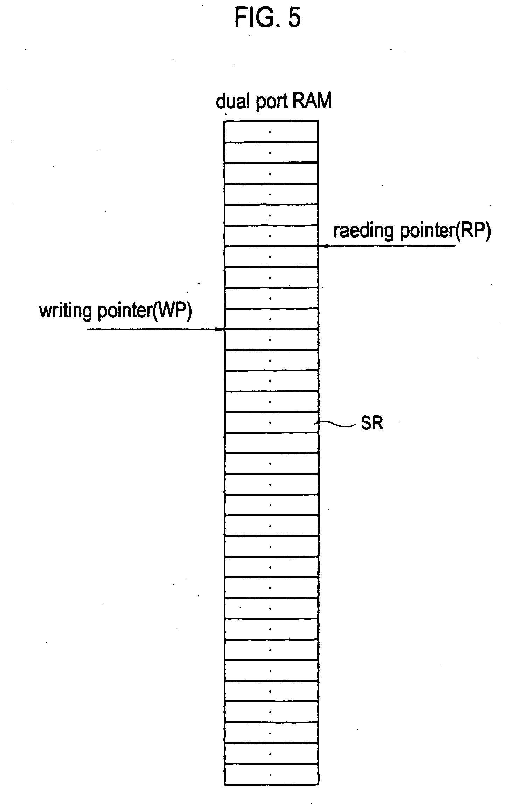

The coarse delaying unit 131 may be configured with a dual-port random access memory (RAM). Referring to FIG. 5, the dual-port RAM includes a plurality of storing regions SRs, a writing pointer WP, a reading pointer RP, a writing pin (not shown) and a reading pin (not shown). The number of storing regions SRs is not more than that of probe elements. Digital signals inputted through the writing pin are stored in the storing region pointed by the writing pointer WP. Further, digital signals stored in the storing region pointed by the reading pointer RP are outputted through the reading pin. Both the writing and reading pointers of the DPR indicate the same storing region at an initial state. The initial state means that the digital signals have not been outputted from any of the ADCs 120. After a predetermined time from storing the digital signals in each region or from pointing each region with the writing pointer, the reading pointer points the storing region. The predetermined time is based on a delay profile, which reflects the distance differences between the probe elements and the target object.

The extracting unit 132 controls the amount of coarsely-delayed digital signals based on the center frequency of the analog receiving signal outputted from the probe elements. The extracting unit 132 includes a shift register 132a and a processing register 132b. The numbers of the shift register 132a and the processing register 132b are equal to that of the number of receiving channel of system. If multiple receiving scan lines are formed by the time divisional multiplexing, the numbers of the shift register 132a and the processing register 132b increase in proportion to the number of the multiple receiving scan lines. The shift register 132a receives the digital signals in the storing region pointed by the reading pointer RP under the control of the controlling unit 134. Parts of the coarsely-delayed digital signals stored in the shift register 132a are extracted at an extraction rate of n-times of the center frequency. The extracted digital signals are moved to the processing register 132b.

The extracting unit 132 extracts parts of the coarsely delayed digital signals at a rate DR, which is defined as the following equation 1.

$$DR = n \times fc \quad \text{............................................(1)}$$

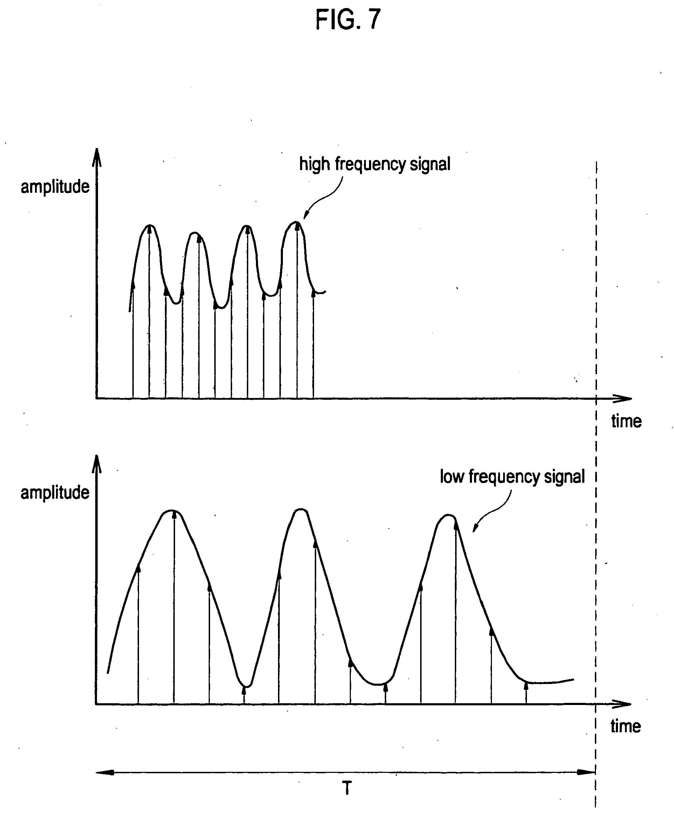

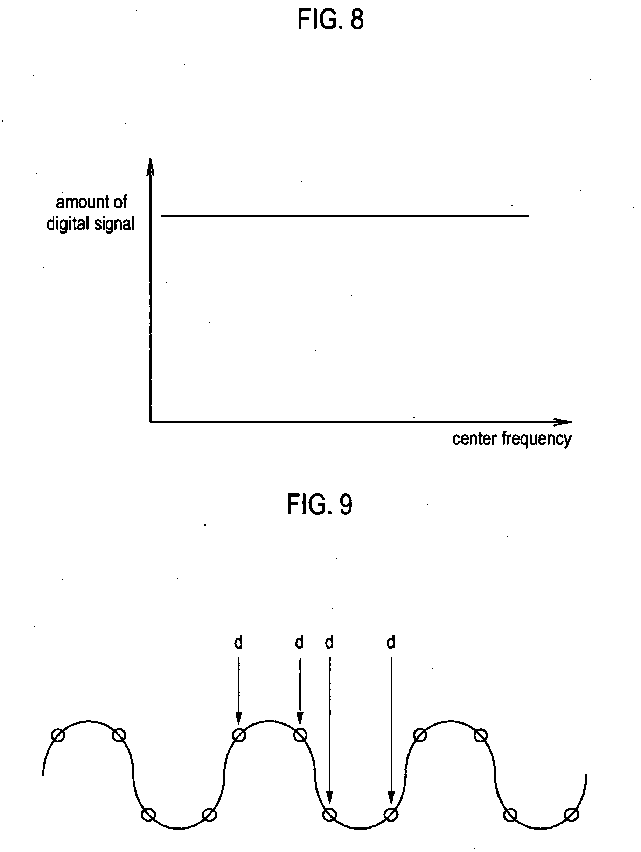

In equation 1, "fc" and "n" denote the center frequency and the positive integer, respectively. If it is guaranteed that bandwidth is two times of the center frequency of the analog receiving signals, the highest frequency becomes two times of the center frequency (2fc). In order to reduce aliasing, the sampling rate, i.e., the extraction rate must be twice the highest analog frequency component (at least $2f_{max}$) according to the Nyquist Theorem. As mentioned above, the coarsely delayed digital signals are extracted at the rate of n-times of the center frequency. Therefore, the coarsely delayed digital signals are extracted at a much higher extraction rate than the constant sampling rate of the ADC 120, in case that the analog receiving frequency is high (i.e., less digital signals are outputted from the ADC 120 per one cycle). Also, the coarsely delayed digital signals are extracted at a much lower extraction rate than the constant sampling rate of the ADC 120, in case that the analog receiving frequency is low (i.e., much digital signals are outputted from the ADC 120 per one cycle). For example, as shown in FIG. 7, if the center frequency is high, the digital signals are extracted at a higher extraction rate compared to the constant sampling rate of the ADC 120. If the center frequency is low, the digital signals are extracted at a lower extraction rate compared to the constant sampling rate of the ADC 120 (In FIG. 7, the high and low frequency signals are shown in analog waveforms, although they have digital waveforms). Therefore, it is possible to compensate excessive digital signals from being outputted by not using relatively high extraction rate when the center frequency is relatively low. Also, it is possible to compensate deficient digital signals by not using relatively low extraction rate when the center frequency is relatively high. Therefore, the beam-former may output a nearly uniform amount of digital signals, regardless of the amount of digital signals outputted from the ADC 120 per one cycle, as shown in FIG. 8. Attenuation degree of the signals is proportion to the frequency, and the visible depth (or penetration depth) is reduced as the frequency increases. In general, the maximum visible depth is nearly 512 time of the wavelength, which is defined with the center frequency, and the amount of the digital signals outputted from the ADC directly depends on the center frequency. However, in the present invention, the amount of the extracted digital signals per one cycle, is controlled with the extraction rate defined as equation 1, which is determined in consideration of the relation between the center frequency and attenuation degree. Therefore, relatively uniform amount of the extracted digital signals can be obtained regardless of the center frequency size.

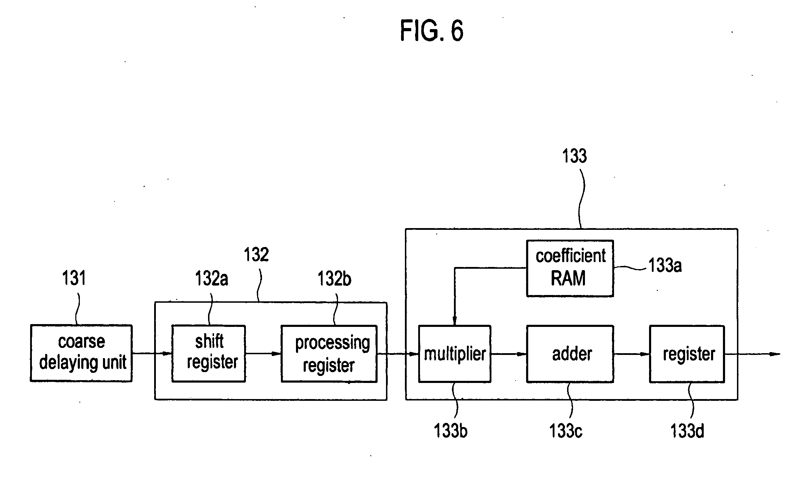

The interpolating unit 133 performs interpolation with the digital signals outputted from the processing register 132b. As shown in FIG. 6, the interpolating unit 133 includes a coefficient RAM 133a, a multiplier 133b, an adder 133c and a register 133d. The coefficient RAM 133a provides a look-up table of filter coefficients. In interpolating, the multiplier 133b multiplies the interpolation filter coefficients to the extracted digital signals inputted from the processing register 132b. Further, the adder 133c adds the outputs of the multipliers 133b. The outputs of the adder 133b, i.e., focused-receiving signals, are stored in the register 133d.

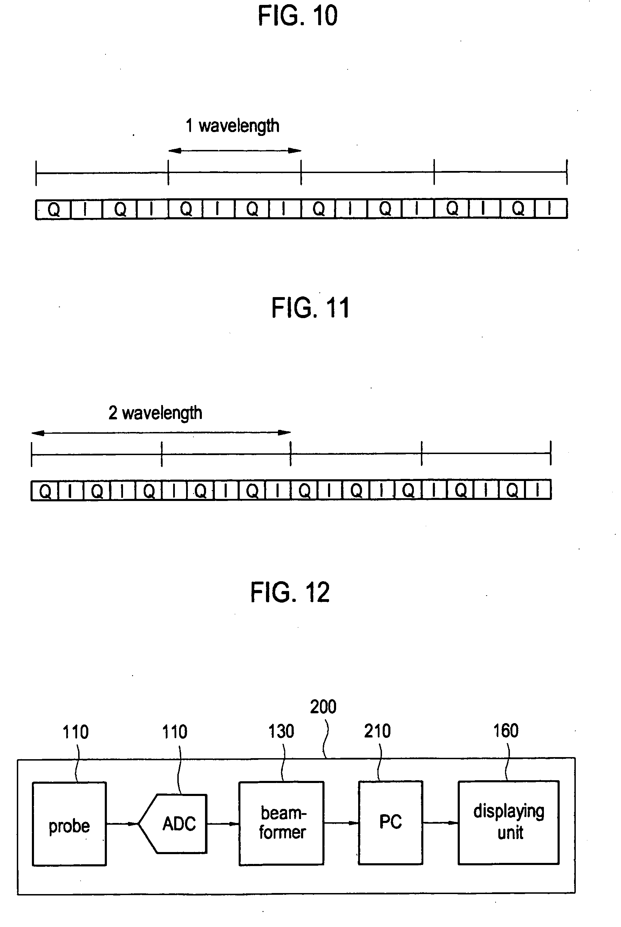

DSP 140 forms image data (i.e., IQ data) with the focused-receiving signal outputted from the beam-former 130. The IQ data are used to form an ultrasound image of the A, B, C, M or D mode. Specifically, the DSP 140 selects at least one pair of signals from the focused-receiving signal outputted from the beam-former 130 at the rate of n-times of the center frequency to form the IQ data. In each pair, the focused-receiving signals have a phase difference of λ/4 with respect to each other. For example, the DSP 140 may select signals d (d1, d2, d3, and d4) at a rate of 4-times of the center frequency, as shown in FIG. 9. The signals d1 and d2 have a phase difference of $\lambda/4$ with respect to each other, and the signals d2 and d3, d3 and d4, also have a phase difference of $\lambda/4$ relative to each other. If the beam-former 130 outputs signals d1, d2, d3, d4, d5, d6, d7, d8 and so on, then signals d1, -d3, d5, -d7... are stored in a memory equipped for Q data and data d2, -d4, d6, -d8... are stored in another memory equipped for I data. The IQ data can be formed with pairs of (d1, d2), (-d3, -d4), (d5, d6), (-d7, -d8) and so on. FIGS. 10 and 11 show a relationship between the number of IQ data and the wavelength of the center frequency.

The signals of each pair for forming the IQ data are not selected at the same time. Thus, some processes should be performed to compensate for the time difference. These processes may be performed with the coefficient of the interpolation filter for a fine delay. Hereinafter, the focused receiving signals of each pair should be selected at the same time. In an embodiment of the present invention, the DSP 140 may include a compensator instead of the interpolation filter for compensating the selection time differences of the focused receiving signals.

If it is supposed that the focused receiving signals of each pair are selected at the same time, new signal pairs for forming IQ data may be obtained by combining the pairs. For an instance, a new pair, i.e., (d1-d3, d2-d4) may be formed with the one pair (d1, d2) and (-d3, -d4).

The DSC 150 scan-converts the image data inputted from the DSP 140. The displaying unit 160 then displays an ultrasound image with the scan-converted image data.

Referring to FIG. 12, an ultrasound diagnostic system constructed in accordance with another embodiment of the present invention may include a personal computer (PC) instead of the DSP 140 and DSC 150. In the PC, a program for performing the functions of the DSP 140 and DSC 150 is installed.

In accordance with a method of the present invention, ultrasound echoes from a target object are converted into analog signals, which have a center frequency. The analog signals are then converted into digital signals. Parts of the digital signals are extracted at a rate of n-times of the center frequency. Focused-receiving signals are formed with the extracted digital signals. IQ data are obtained by selecting at least one pair of the focused-receiving signals, which have a phase difference of $\lambda/4$ with respect to each other.

According to the embodiments of the present invention, it is possible to obtain the IQ data without the quadrature demodulator. Therefore, the ultrasound diagnostic system can be designed without any limitation or restriction caused by the quadrature demodulator. Further, the beam-former outputs a relatively uniform amount of focused-receiving signals. Thus, the process capacity of an image processor, such as the DSP and the PC, may not be considered in designing the system.

An ultrasound diagnostic system for forming IQ data without a quadrature demodulator is disclosed. This system includes: a probe for receiving ultrasound echoes from a target object and outputting analog signals by converting the ultrasound echoes, wherein the analog signals have a center frequency; an analog-digital converter for converting the analog signals into digital signals; a beam-former for extracting parts of the digital signals at a rate of n-times of the center frequency and forming focused-receiving signals with the extracted digital signals, wherein "n" is a positive integer; and a digital signal processing unit for forming IQ data by selecting at least two focused-receiving signals, wherein the selected focused-receiving signals have a phase difference $\lambda/4$ with respect to each other, wherein " $\lambda$" is a wavelength, which is defined with the center frequency of analog signals.

Also, a method of forming IQ data without a quadrature demodulator is disclosed. This method includes: converting ultrasound echoes from a target object into analog signals, wherein the analog signals have a center frequency; converting the analog signals into digital signals; extracting parts of the digital signals at a rate of n-times of the center frequency, wherein "n" is a positive integer; and forming focused-receiving signals with the extracted digital signals; forming IQ data by selecting at least two pairs of focused-receiving signals, wherein the selected focused-receiving signals have a phase difference of $\lambda/4$ with respect to each other, wherein " $\lambda$" is a wavelength, which is defined with the center frequency of the analog signals.

**Claims**

1. An ultrasound diagnostic system, comprising:

   a probe (110) for receiving ultrasound echoes from a target object and outputting analog signals by converting the ultrasound echoes, wherein the analog signals have a center frequency;
   an analog-digital converter (120) for converting the analog signals into digital signals;
   a beam-former (130) for extracting parts of the digital signals at a rate of n-times of the center frequency and forming focused-receiving signals with the extracted digital signals, wherein "n" is a positive integer; and
   a digital signal processing unit (140) for forming IQ data by selecting at least two focused-receiving signals, wherein the selected focused-receiving signals have a phase difference of $\lambda/4$ with respect to each other, wherein " $\lambda$" is a wavelength determined with the center frequency.

**2.** The system of Claim 1, wherein the digital signal processing unit selects at least two focused receiving signals per one cycle, wherein the cycle is defined with the center frequency of the analog signals.

**3.** The system of Claim 1, wherein the digital signal processing unit includes:

a compensator for compensating selection time differences of the focused receiving signals in said each pair.

**4.** The system of Claim 1, wherein the beam-former includes:

an extracting unit (132) for controlling an amount of the digital signals by extracting the parts of the digital signals at the rate of the n-times of the center frequency.

**5.** The system of Claim 4, wherein the extracting unit includes:

a register (132a) for storing the digital signals inputted from the analog digital converter; and
a processing register (132b) for storing the extracted digital signals.

**6.** The system of Claim 5, wherein the beam-former further includes:

an interpolating unit (133) for performing interpolation with the extracted digital signals.

**7.** The system of Claim 6, wherein the interpolating unit includes:

a coefficient RAM (133a) for providing a look-up table of filter coefficients;
a multiplier (133b) for multiplying the filter coefficients to the extracted digital signals; and
an adder (133c) for forming the focused receiving signals by adding outputs of the multipliers.

**8.** The system of Claim 4, wherein the probe includes a plurality of probe elements, and wherein the beam-former further includes a delaying unit (131) for delaying the digital signals inputted from the analog-digital converter in consideration of distances between the probe elements and the target object.

**9.** The system of Claim 8, wherein the delaying unit is configured with a dual port RAM.

**10.** A method of forming IQ data, comprising:

converting ultrasound echoes from a target object into analog signals, wherein the analog signals have a center frequency;
converting the analog signals into digital signals;
extracting parts of the digital signals at a rate of n-times of the center frequency, wherein "n" is a positive integer; and
forming focused-receiving signals with the extracted digital signals;
forming IQ data by selecting at least one pair of focused-receiving signals, wherein the selected focused-receiving signals have a phase difference of $\lambda/4$ with respect to each other, wherein " $\lambda$" is a wavelength determined with the center frequency.

**11.** The method of Claim 10, further comprising:

compensating selection time differences between the focused receiving signals in said each pair.

**12.** The method of Claim 11, said at least two focused receiving signals are selected per one cycle, wherein the cycle is defined with the center frequency of the analog signals.

**Patentansprüche**

**1.** Ultraschalldiagnosesystem, welches Folgendes aufweist:

einen Messfühler (110) zum Empfangen von Ultraschallechos von einem Zielobjekt und Ausgeben von analogen Signalen durch Umwandeln der Ultraschallechos, wobei die analogen Signale eine Mittenfrequenz aufweisen; einen Analog-Digital-Konverter (120) zum Konvertieren der analogen Signale in digitale Signale; einen Strahlformer (130) zum Extrahieren von Teilen der digitalen Signale mit einer Geschwindigkeit von dem n-fachen der Mittenfrequenz und zum Bilden von fokussierten Empfangssignalen aus den extrahierten digitalen Signalen, wobei "n" eine positive ganze Zahl ist; und

eine digitale Signalverarbeitungseinheit (140) zum Bilden von IQ-Daten durch Auswählen von wenigstens zwei fokussierten Empfangssignalen, wobei die ausgewählten fokussierten Empfangssignale eine Phasenverschiebung von λ/4 in Bezug zueinander aufweisen, wobei "λ" eine mit der Mittenfrequenz ermittelte Wellenlänge ist.

2. System nach Anspruch 1, wobei die digitale Signalverarbeitungseinheit wenigstens zwei fokussierte Empfangssignale pro Zyklus auswählt, wobei der Zyklus mit der Mittenfrequenz der analogen Signale definiert ist.

3. System nach Anspruch 1, wobei die digitale Signalverarbeitungseinheit Folgendes aufweist:

   einen Kompensator zum Kompensieren von AuswahlZeitunterschieden der fokussierten Empfangssignale in jedem Paar.

4. System nach Anspruch 1, wobei der Strahlformer Folgendes aufweist:

   eine Extraktionseinheit (132) zum Steuern einer Menge der digitalen Signale durch Extrahieren der Teile der digitalen Signale mit der Geschwindigkeit von dem n-fachen der Mittenfrequenz.

5. System nach Anspruch 4, wobei die Extraktionseinheit Folgendes aufweist:

   ein Verzeichnis (132a) zum Speichern der von dem Analog-Digital-Konverter eingegebenen digitalen Signale; und
   ein Verarbeitungsverzeichnis (132b) zum Speichern der extrahierten digitalen Signale.

6. System nach Anspruch 5, wobei der Strahlformer des weiteren Folgendes aufweist:

   eine Interpoliereinheit (133) zum Durchführen einer Interpolation mit den extrahierten digitalen Signalen.

7. System nach Anspruch 6, wobei die Interpoliereinheit Folgendes aufweist:

   ein Koeffizienten-RAM (133a), um eine Nachschlagetabelle von Filterkoeffizienten zur Verfügung zu stellen;
   einen Multiplikator bzw. Vervielfacher (133b) zum Vervielfachen der Filterkoeffizienten zu den extrahierten digitalen Signalen; und
   einen Addierer (133c) zum Bilden der fokussierten Empfangssignale durch Addieren von Ausgaben der Vervielfacher.

8. System nach Anspruch 4, wobei der Fühler eine Vielzahl von Fühlerelementen aufweist, und wobei der Strahlformer eine Verzögerungseinheit (131) zum Verzögern der von dem Analog-Digital-Konverter eingegebenen digitalen Signale unter Berücksichtigung von Abständen zwischen den Fühlerelementen und dem Zielobjekt aufweist.

9. System nach Anspruch 8, wobei die Verzögerungseinheit mit einem RAM mit doppeltem Eingang ausgebildet ist.

10. Verfahren zur Bildung von IQ-Daten, welches Folgendes aufweist:

    Konvertieren von Ultraschallechos von einem Zielobjekt in analoge Signale, wobei die analogen Signale eine Mittenfrequenz aufweisen;
    Konvertieren der analogen Signale in digitale Signale;
    Extrahieren von Teilen der digitalen Signale mit einer Geschwindigkeit von dem n-fachen der Mittenfrequenz, wobei "n" eine positive ganze Zahl ist; und
    Bilden von fokussierten Empfangssignalen mit den extrahierten digitalen Signalen;
    Bilden von IQ-Daten durch Auswählen von wenigstens einem Paar von fokussierten Empfangssignalen, wobei die ausgewählten fokussierten Empfangssignale eine Phasenverschiebung von λ/4 im Bezug zueinander aufweisen, wobei "λ" eine mit der Mittenfrequenz ermittelte Wellenlänge ist.

**11.** Verfahren nach Anspruch 10, welches des weiteren Folgendes aufweist:

Kompensieren von Auswahlzeitunterschieden zwischen den fokussierten Empfangssignalen in jedem Paar.

**12.** Verfahren nach Anspruch 11, wobei die wenigstens zwei fokussierten Empfangssignale in jedem Zyklus ausgewählt werden, wobei der Zyklus mit der Mittenfrequenz der analogen Signale festgelegt wird.

**Revendications**

**1.** Système de diagnostic par ultrasons comprenant :

une sonde (110) pour recevoir des échos d'ultrasons d'un objet cible et émettant des signaux analogiques en convertissant les échos d'ultrasons, dans lequel les signaux analogiques ont une fréquence centrale ;
un convertisseur analogique-numérique (120) pour convertir les signaux analogiques en signaux numériques ;
un élément de mise en forme de faisceau (130) pour extraire des parties des signaux numériques à un taux de n-fois la fréquence centrale et pour former des signaux de réception focalisés à partir des signaux numériques extraits, dans lequel "n" est un entier positif ; et
une unité de traitement du signal numérique (140) pour former des données IQ en sélectionnant au moins deux signaux de réception focalisés, dans lequel les signaux de réception focalisés sélectionnés ont une différence de phase de λ/4 l'un par rapport à l'autre, dans lequel "λ" est une longueur d'onde définie par la fréquence centrale.

**2.** Système de la revendication 1, dans lequel l'unité de traitement du signal numérique sélectionne au moins deux signaux de réception focalisés par cycle, dans lequel le cycle est défini par la fréquence centrale des signaux analogiques.

**3.** Système de la revendication 1, dans lequel l'unité de traitement du signal numérique comprend :

un compensateur pour compenser les différences de temps de sélection des signaux de réception focalisés dans ladite chaque paire.

**4.** Système de la revendication 1, dans lequel l'élément de mise en forme de faisceau comprend :

une unité d'extraction (132) pour contrôler une quantité des signaux numériques en extrayant les parties des signaux numériques à un taux de n-fois la fréquence centrale.

**5.** Système de la revendication 4, dans lequel l'unité d'extraction comprend :

un registre (132a) pour stocker les signaux numériques reçus du convertisseur analogique-numérique et un registre de traitement (132b) pour stocker les signaux numériques extraits.

**6.** Système de la revendication 5, dans lequel l'élément de mise en forme de faisceau comprend :

une unité d'interpolation (133) pour effectuer une interpolation avec les signaux numériques extraits.

**7.** Système de la revendication 6, dans lequel l'unité d'interpolation comprend :

une RAM de coefficients (133a) pour fournir un tableau de consultation de coefficients de filtre ;
un multiplicateur (133b) pour multiplier les signaux numériques extraits par les coefficients de filtre ; et
un additionneur (133c) pour former les signaux de réception focalisés en additionnant les sorties des multiplicateurs.

**8.** Système de la revendication 4, dans lequel la sonde comprend une pluralité d'éléments de sonde, et dans lequel l'élément de mise en forme de faisceau comprend en outre une unité de retard (131) pour retarder les signaux numériques reçus du convertisseur analogique-numérique en tenant compte des distances entre les éléments de sonde et l'objet cible.

**9.** Système de la revendication 8, dans lequel l'unité de retard est configurée avec une RAM à porte double.

**10.** Procédé pour former des données IQ, comprenant :

la conversion d'échos ultrasons d'un objet cible en signaux analogiques, dans lequel les signaux analogiques ont une fréquence centrale ;

la conversion des signaux analogiques en signaux numériques ;

l'extraction des parties des signaux numériques à un taux de n-fois la fréquence centrale, dans lequel "n" est un entier positif ; et

la formation de signaux de réception focalisés à partir des signaux numériques extraits ;

la formation de données IQ en sélectionnant au moins une paire de signaux de réception focalisés, dans lequel les signaux de réception focalisés sélectionnés ont une différence de phase de $\lambda/4$ l'un par rapport à l'autre, dans lequel "$\lambda$" est une longueur d'onde définie par la fréquence centrale.

**11.** Procédé de la revendication 10, comprenant en outre :

la compensation des différences de temps de sélection entre les signaux de réception focalisés dans ladite chaque paire.

**12.** Procédé de la revendication 11, lesdits au moins deux signaux de réception focalisés étant sélectionnés par cycle, dans lequel le cycle est défini par la fréquence centrale des signaux analogiques.

# FIG. 1

<u>10</u>

| 11 | 12 | 13 | 14 | 15 |
|---|---|---|---|---|
| probe | beam-former | scan line data forming unit | DSC | displaying unit |

# FIG. 2

Quadrature demodulator

COS(2 * pi * fc * t)

Focused RF data → high pass filter (13a) → 13b ⊗ → LPF (13d) → I → memory (13f) → scan line date

13c ⊗ → LPF (13e) → Q

sin(2 * pi * fc * t)

## FIG. 3

```
                                 100
  110        120        130   (    140        150        160
   |          |          |           |          |          |

┌──────┐   ┌─────┐  ┌────────┐  ┌──────┐   ┌──────┐   ┌──────────┐
│probe │──▶│ ADC │─▶│ beam-  │─▶│ DSP  │──▶│ DSC  │──▶│displaying│
│      │   │     │  │ former │  │      │   │      │   │   unit   │
└──────┘   └─────┘  └────────┘  └──────┘   └──────┘   └──────────┘
```

## FIG. 4

```
                                              130
  120          131           132     (        133
   |            |             |                |

┌─────┐   ┌───────────┐  ┌──────────┐   ┌─────────────┐
│ ADC │──▶│  coarse   │─▶│extracting│──▶│interpolating│
│     │   │delaying   │  │   unit   │   │    unit     │
└─────┘   │  unit     │  └──────────┘   └─────────────┘
          └───────────┘        │
                    │    ┌────────────┐
                    └───▶│ controlling│
                         │    unit    │
                         └────────────┘
                               |
                              134
```

# FIG. 5

dual port RAM

raeding pointer(RP)

writing pointer(WP)

SR

FIG. 6

EP 1 860 461 B1

```
                                                                    133
                                                                     │
                                                        ┌──────────────────────┐
                                                        │          ┌──────────┐│
                                                        │          │coefficient││──133a
                                                        │          │  RAM     ││
                                                        │          └────┬─────┘│
  131         132a       132          132b      133b    │       133c      133d │
   │            │         │            │          │      │        │         │   │
┌─────────┐  ┌────────┐     ┌──────────┐    ┌──────────┐  ┌──────────┐  ┌──────────┐
│ coarse  │  │ shift  │     │processing│    │multiplier│  │  adder   │  │ register │ ──→
│delaying │→ │register│  →  │ register │ →  │          │→ │          │→ │          │
│  unit   │  │        │     │          │    │          │  │          │  │          │
└─────────┘  └────────┘     └──────────┘    └──────────┘  └──────────┘  └──────────┘
```

EP 1 860 461 B1

# FIG. 7

14

# FIG. 8

amount of
digital signal

center frequency

# FIG. 9

d      d   d     d

## FIG. 10

1 wavelength

| Q | I | Q | I | Q | I | Q | I | Q | I | Q | I | Q | I | Q | I |

## FIG. 11

2 wavelength

| Q | I | Q | I | Q | I | Q | I | Q | I | Q | I | Q | I | Q | I | Q | I | Q | I |

## FIG. 12

200

110    110    130    210    160

| probe | → | ADC | → | beam-former | → | PC | → | displaying unit |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RANGANATHAN, K.** A Prototype Low Cost Handheld Ultrasound Imaging System. *Proc. of SPIE,* 2004, vol. 5373, 24-32